(19) Europäisches Patentamt
European Patent Office
Office européen des brevets

(11) Publication number: 0 153 881
A2

# (12) EUROPEAN PATENT APPLICATION

(21) Application number: 85301439.7

(22) Date of filing: 01.03.85

(51) Int. Cl.4: A 61 K 35/78

(30) Priority: 02.03.84 US 585374

(43) Date of publication of application:
04.09.85 Bulletin 85/36

(84) Designated Contracting States:
AT BE CH DE FR GB IT LI LU NL SE

(71) Applicant: THE JOHNS HOPKINS UNIVERSITY
34th and Charles Streets
Baltimore, MD 21218(US)

(72) Inventor: Lichtenstein, Lawrence M.
1600 The Terraces
Baltimore Maryland, 21209(US)

(72) Inventor: Pickett, Walter C.
15 Brookside Avenue
Suffern New York, 10901(US)

(74) Representative: Arthur, Bryan Edward et al,
Withers & Rogers 4 Dyer's Buildings Holborn
London EC1N 2JT(GB)

## (54) Treatment of allergies and inflammatory conditions.

(57) Garlic oil or onion oil, preferably certain fractions or extracts thereof, are used to inhibit histamine release.

EP 0 153 881 A2

# TREATMENT OF ALLERGIES AND INFLAMMATORY CONDITIONS

The present invention relates to the treatment of allergies and inflammatory conditions. More particularly the invention is concerned with the use of garlic oil or onion oil, or extracts thereof, to inhibit basophil and mast cell mediators which cause human allergic disease (hereinafter referred to for convenience as "histamine release").

It is now well known that histamine leucotrienes and other mediators are the cause of certain allergies or allergic reactions, e.g. asthma, allergic rhinitis, skin disorders and the like. The invention is based on the finding that garlic and onion oils, and especially certain extracts therefrom, are highly effective in inhibiting histamine release and, therefore, should be useful in treating histamine release-dependent allergies.

The extracts of the invention are obtained by the fractionation of garlic or onion oil using high pressure liquid chromatography (HPLC). Certain aspects of the HPLC fractionation to obtain the present extracts are also new in the context of the fractionation involved and constitute further features of the invention.

An abstract published in The Journal of Allergy and Clinical Immunology, Volume 73, page 147, January, 1984, by Dorsch et al refers to the depression or reduction of immediate and late allergic skin reactions (LCR) by means of alcoholic onion extract and suggests that onions might contain possible antiallergic resp. antiinflammatory substances. The authors refer to an earlier paper (J. Allergy Clin. Immunol. 72:168, 1983) as reporting that a selective thromboxane synthetase blocker can inhibit LCR in man. They also refer to a paper by Vanderhoek, Biochem. Pharmacol. 29:3169 (1980) as showing that onion extracts exert inhibitory effects in vitro upon the biosynthesis of thromboxanes, as well as certain prostaglandins and some lipoxygenase products. The work reported in the abstract by Dorsch et al is, therefore, based on the view that onion extract might be effective against LCR by blocking thromboxane synthesis. The present invention in contrast is based on the concept that garlic and onion oils, and especially certain extracts thereof as described herein, function by inhibiting histamine release and by inhibiting leukotriene $B_4$($LTB_4$) and $C_4$($LTC_4$) release which are themselves involved in the mechanism of histamine release.

The above-mentioned paper by Vanderhoek (Biochem. Pharmacol. 29:3169-3173) describes the inhibiting effect of onion and garlic oils on platelet aggregation or fatty acid oxygenases. The effects of garlic extract and of three components isolated from it on platelet aggregation, arachidonate metabolism, release reaction and platelet ultrastructure are also discussed in a paper by Apitz-Castro et al, Thrombosis Research 32; 155-169, Pergamon Press, 1983. Neither of these papers discloses that onion or garlic oil or extracts therefrom might be useful as inhibitors of histamine release.

As noted above, the extracts of the invention are obtained by fractionation of onion oil or garlic oil using HPLC. Garlic and onion oils are commercially available (see, for example, the Vanderhoek paper referred to above) and any such oil may be used for present purposes. In a preferred embodiment, the oil (garlic, Egyptian) is fractionated by HPLC using a mixture of 75% ethanol and 25% water (volume basis) as the mobile phase. The mobile phase, with the oil added thereto, is pumped at a rate of 1.5 ml. per minute at room temperature (about 25°C) using a C18 reverse phase (5 micron spherical particle) semiprep stainless steel column (IBM Instruments Company, Danbury, Conn.). Typically in one run, 180 milligrams (mg.) of the oil is fractionated into 1.5 ml fractions which are then tested for bioactivity, notably for inhibition of histamine release and/or $LTC_4$ release from human mast cells or basophils and inhibition of 5-lipoxygenase ($LTC_4$, $LTB_4$ and 5-HETE) activity in guinea pig neutrophiles. The most effective inhibiting fractions according to the invention are those collected in the 19th-24th minutes of fractionation, particularly the 21-22 minute fractions.

It is an important aspect of the fractionation process that a mixture of ethanol and water or the equivalent is employed as the mobile phase. This mixture provides highly effective separation of the oil components. Additionally, the mixture does not undesirably affect the test cells. It is surprising that the ethanol/water mixture is effective for the fractionation because the oil is not soluble in this mixture and it is normally thought necessary or desirable for effective HPLC to use a mobile phase which is a solvent for the material being fractionated.

The composition of the most effective oil extracts, or the active component thereof, has not as yet been fully defined. As noted, the most active fractions are those obtained in the 21st-22nd minutes of HPLC fractionation. A substantial component of these fractions appears to be diallyltrisulfide.

Activity against histamine release is determined in vitro by well-established techniques using basophils in mixed leukocyte preparations or dispersed human lung mast cells.

Following preparation of the cells (basophils in mixed leukocyte preparations or dispersed human lung mast cells), they are added to individual assay tubes containing buffer (controls) or dilutions of the compound being tested for histamine or $LTC_4$ release inhibition. Approximately 2 x $10^4$ basophils or mast cells per tube are used, the final volume being 1.0 ml. Following a ten-minute preincubation of the cells with the test compound at 37°C, the histamine release reaction is begun by adding the stimulus (antigen or anti-human IgE) to all tubes. A concentration of stimulus selected to release about 30 to 50% of total cellular histamine is used in order to optimize inhibition. Aliquots of cells are lysed with 1.6% perchloric to measure total histamine content and cells are incubated with buffer alone to assess spontaneous release ($\leq$5% of total). After addition of the stimulus, the reaction is allowed to proceed for 45 minutes at 37°C, the cells pelleted and the supernatants assayed for histamine using an automated fluorometric technique sensitive to $\leq$1 ng/ml.

Following subtraction of spontaneous release from all results, percent inhibition is calculated as follows:

$$\frac{\text{Control release minus release with test compound}}{\text{control release}} \times 100$$

After the first assessment of the test compound, controls to correct for lytic effects, drug fluorescence and assay interference are included as necessary.

The above procedure, using basophil leukocytes and lung mast cells, provides a reliable in vitro test for determining the histamine release inhibition properties of a test compound, e.g. an oil extract according to the invention. The test results correlate well with in vivo studies in humans and provide a relatively simple and accurate way of determining the activity of a test compound as an inhibitor of histamine release and predictor for use in treating histamine release-dependent allergies and the like in humans or other warm blooded animals.

The method for preparing basophil leukocytes and lung mast cells for use herein is well known in the art. See, for example, the paper by Peters et al entitled "Dispersed Human Lung Mast Cells", Am. Rev. Respir. Dis. 1982; 126:1034-1039 which describes the preparation of dispersed human lung mast cells and their use in tests to determine histamine release inhibition. Similarly, the preparations and use of human basophils for the determination of histamine inhibition are described by Marone et al, The Journal of Immunology, Vol. 123, No. 4, pp. 1669-1677 (1979). Accordingly, it is not necessary herein to detail the manner in which test cells are prepared for use in determining the histamine release inhibition activity of the extracts of the invention.

The compositions of the invention may be administered orally, topically or parenterally to any warm blooded animal, including man, where there is a need to inhibit histamine release, e.g. in a subject suffering from asthma, rhinitis or other allergy caused by undesired histamine release. The compositions may thus take the form of, for example, orally administrable tablets or capsules, solutions, suspensions or emulsions which are suitable for oral or topical administration, or sterile injectable formulations, all comprising an oil extract according to the invention as the active component and a pharmaceutically acceptable carrier.

The amount of extract used will vary depending on, for example, the mode of administration and the severity of the condition being treated. Broadly speaking, the amount employed should be adequate to attain a blood plasma level in the order of 10-50 micrograms (mcg) or more per milliliter of plasma. This amount should be adequate in most cases to control undesired histamine release.

The invention is illustrated by the following examples:

Example 1

Egyptian garlic oil was subjected to HPLC using a 75/25 ethanol/water mixture as the mobile phase and a C18 reverse phase semiprep stainless steel column as and in the manner described above.

Fractions obtained at 15-16, 17-18, 19-20, 21-22 and 23-24 minutes were collected and tested for histamine release inhibition on basophil cells in the manner described above. The fractions were compared against unfractionated garlic oil as a control at two different test concentrations (80 mcg/ml and 27 mcg/ml). Fractions corresponding to the following elution times, neat or diluted about 3:1 with test buffer i.e. equivalent to 80 mcg/ml and 27 mcg/ml, were found to have the following percent inhibition compared with the controls at equivalent concentrations:

| Fraction | % Inhibition |
|---|---|
| (80 mcg/ml) | |
| 15-16 minutes | 27% |
| 17-18 minutes | 32% |
| 19-20 minutes | 69% |
| 21-22 minutes | 84% |
| 23-24 minutes | 73% |
| (27 mcg/ml) | |
| 15-16 minutes | 2% |
| 17-18 minutes | 2% |
| 19-20 minutes | 38% |
| 21-22 minutes | 27% |
| 23-24 minutes | 27% |
| Control | |
| 80 mcg/ml unfractionated oil | 83% |
| 27 mcg/ml " " | 79% |

It will be noted from the above inhibition values that optimum inhibition is realized for the fractions obtained at 19-24 minutes using the indicated HPLC system. The data also show that the degree of inhibition is dose responsive, the 80 mcg/ml test samples giving a higher degree of inhibitions than the 27 mcg/ml samples.

Those in the art will appreciate that the inhibition figures given above will vary depending, for example, on cell activity or cell donor for the day involved. Thus, the percent inhibition may vary from test day to test day. The tests reported above were conducted on the same day and even though lower or higher inhibition rates might be realized on another day, the relative activities for the different fractions should remain the same.

Example 2

This example illustrates the effect of garlic oil and fractions thereof on neutrophil lipoxygenase (5-HETE and/or $LTB_4$) activity. Five fractions obtained by HPLC fractionation as described above and representing the 15-16, 17-18, 19-20, 21-22 and 23-24 minute fractions were subjected to the lipoxygenase assay. In this assay, 3 x $10^7$ peritoneal neutrophils derived from guinea pig were incubated at 37°C in Dulbeccos buffer containing 50 mM tris (pH 7.4). Five minutes before the addition of 100 micromolar arachidonic acid and 20 micromolar calcium ionophore (A23187) control vehicle, garlic oil or the fraction thereof was added to the neutrophil. Three minutes after the addition of arachidonic acid and calcium ionophore, 5-hydroxyeicosatetraenoic acid and leukotriene $B_4$ were extracted and measured to determine inhibition of lipoxygenase activity. The five fractions showed the following inhibition activity against lipoxygenase:

| Fraction | Percent Inhibition |
|---|---|
| 15-16 minutes | 0 |
| 17-18 minutes | 38 |
| 19-20 minutes | 50 |
| 21-22 minutes | 61 |
| 23-24 minutes | 37 |

It will be seen that the 15-16 minutes fraction was essentially inactive as an inhibitor of lipoxygenase. The other four fractions were active with the most effective fraction obtained in the 21-22 minute range.

The graph constituting Figure 1 illustrates the results obtained with various garlic oil fractions separated by HPLC as described above, in terms of inhibition of histamine release and neutrophil lipoxygenase (5-HETE). More particularly, Figure 1 shows the inhibition of histamine released from basophils (mixed leukocytes) stimulated with antigen, and 5 hydroxyeicosatetraeonic acid (5-HETE) biosynthesis from Guinea pig neutrophils incubated with exogenous arachidonic acid and A23187 (calcium ioniphore), inhibition of 5-HETE biosynthesis being a function of lipoxygenase inhibition. The broken line represents the separation of the garlic components.

In Figure 1, the Y-axis indicates the percent inhibition of histamine release, lipoxygenase or relative absorbance. The X-axis indicates the fraction number identified by the minutes after injection of the oil onto the HPLC column.

As shown, the garlic components eluting from the column at 21 minutes demonstrate the best inhibition of histamine release and lipoxygenase activity. This is compared with the unfractionated oil (single points on the right side of the figure).

Figure 2 provides further information regarding garlic oil and medication for allergies and other inflammation conditions. The figure illustrates the various pathways for the metabolism of arachidonic acid through which allergies may arise. The present invention is based on the discovery that garlic and onion oils, and notably certain extracts thereof, effectively inhibit the 5-lipoxygenase and thus undesired histamine release. See the leftmost pathway shown in Figure 2. Vanderhoek et al, in the paper referred to earlier herein, relate to inhibition of the other enzymes shown in Figure 2. None of these appears to be relevant to leukotriene biosynthesis or histamine release.

Example 3

This example and the related Figures 3-6 illustrate the inhibition results obtained using garlic oil and onion oil without fractionation.

100 milligrams (100,000 micrograms) of crude garlic oil were dissolved in alcohol (ethanol). The oil dissolved in alcohol was diluted in a buffer (PAGCM) which is a solution similar to saline except that it is maintained at a neutral pH similar to that in blood (pH 7.4). The basophil leukocytes (i.e. white blood cells which contain histamine and make $LTC_4$) were also diluted in this buffer. The basophils were evenly divided among test tubes containing various amounts of the oil-buffer solutions. The number of micrograms of the oil that were in the test tubes with the basophils is shown on the Y axis of Figures 3 and 4. After the oil was allowed to interact with the basophils for 10 minutes at 37°C (body temperature), the substance (e.g. anti-human IgE) which causes the basophils to release (secrete) histamine and to make $LTC_4$ was added. Without this substance, no histamine or $LTC_4$ is released from the basophils. Some test tubes contained only basophils and the histamine releasing substance; others contained 200, 50, 10, 2 or 0.5 micrograms of oil per milliliter of volume in the test tubes. The test tubes containing oil, basophils and the release substance were maintained at 37°C for 45 minutes so that the maximal possible release could occur. If a substance, such as the oil, inhibits, or lessens, the release of histamine or $LTC_4$, then the amount of these substances in tubes containing the oil is lower than the amount in the tubes containing only the substance which causes release. The percentage of this inhibition is indicated on the X axis of Figures 3 and 4.

After the 45-minute incubation, the test tubes were placed in a centrifuge. The cells went to the bottom of the tubes leaving a clear buffer layer above them. These upper layers, which contain the histamine and $LTC_4$, were poured into new test tubes and the cells were discarded. The amount of histamine in each tube was determined using a fluorometric assay system. The amount of $LTC_4$ in each test tube was measured using a radioimmunoassay, in which an antibody which only attached to $LTC_4$ was used.

The following calculations were then made:

$$\frac{\text{Percent Release with Releasing Substance Alone Minus Percent Release with Oil Present}}{\text{Percent Release with Releasing Substance Alone}} \times 100$$

The curves shown in Figures 3 and 4 were constructed by plotting the percent inhibition of release versus the amount of oil required for this amount of inhibition. As will be evident, Figure 3 shows the percent of inhibitors of histamine release using various concentrations of garlic oil while Figure 4 plots the percent inhibition of leucotriene $C_4$ release versus garlic oil concentration in micrograms per milliliter.

The foregoing test procedure was repeated using onion oil at different concentrations with the inhibition results shown in Figures 5 and 6.

<u>Example 4</u>

The test procedure of Example 3 was repeated using mast cells in place of basophils. The percent inhibition of histamine release for garlic oil and onion oil at different concentrations is shown in Figures 7 and 8.

It will be appreciated from the foregoing that garlic oil, onion oil and various extracts of these oils effectively function to inhibit histamine release and leukotriene release. As a consequence, these oils, and preferably concentrates or extracts of the active components therein, should be useful in treating allergies and inflammatory conditions caused by histamine and/or leukotriene release. A particularly advantageous feature of the invention is that garlic and onion oils are natural products which are not toxic. Hence these oils, and extracts or concentrates thereof, are believed to be suitable for use in treating allergic disorders in general.

Various modifications may be made in the invention described herein. The scope of the invention is defined in the following claims wherein:

WHAT IS CLAIMED IS:

1. A process for inhibiting mediators which cause allergic disease which comprises administering an effective amount of garlic oil, onion oil or extract thereof.

2. A process according to claim 1 for inhibiting undesired histamine release which comprises administering to a warm-blooded animal in need of such inhibition, a histamine release inhibiting amount of garlic oil or onion oil or extract thereof.

3. The process of claim 2 wherein the oil is garlic oil.

4. The process of claim 2 wherein the oil is onion oil.

5. The process of claim 2 wherein an extract is used which corresponds with the fraction obtained by HPLC after garlic oil has been processed in a time range of 19-24 minutes using an ethanol/water mixture at a flow rate of 1.5 ml per minute at room temperature.

6. The process of claim 5 wherein the fraction is the one obtained at 21-22 minutes.

7. A pharmaceutical composition for use in inhibiting histamine release which comprises a histamine release inhibiting amount of garlic oil, onion oil or extract thereof and a pharmaceutically acceptable carrier therefor.

8. The composition of claim 7 in orally administrable form.

9. The composition of claim 7 in topically administrable form.

10. An onion or garlic oil extract corresponding to that obtained at a time range of 19-24 minutes by HPLC fractionation of the oil using an ethanol/water mixture at a flow rate of 1.5 ml per minute at room temperature.

11. A process for fractionating onion or garlic oil which comprises subjecting the oil to HPLC fractionation using an ethanol/water mixture as the mobile phase.

12. The process of claim 11 wherein the volume ratio of ethanol to water is about 3:1.

13. The process of claim 1 when used for treating asthma or other allergy or inflammatory disease caused by undesired histamine release.

# FIG. 1

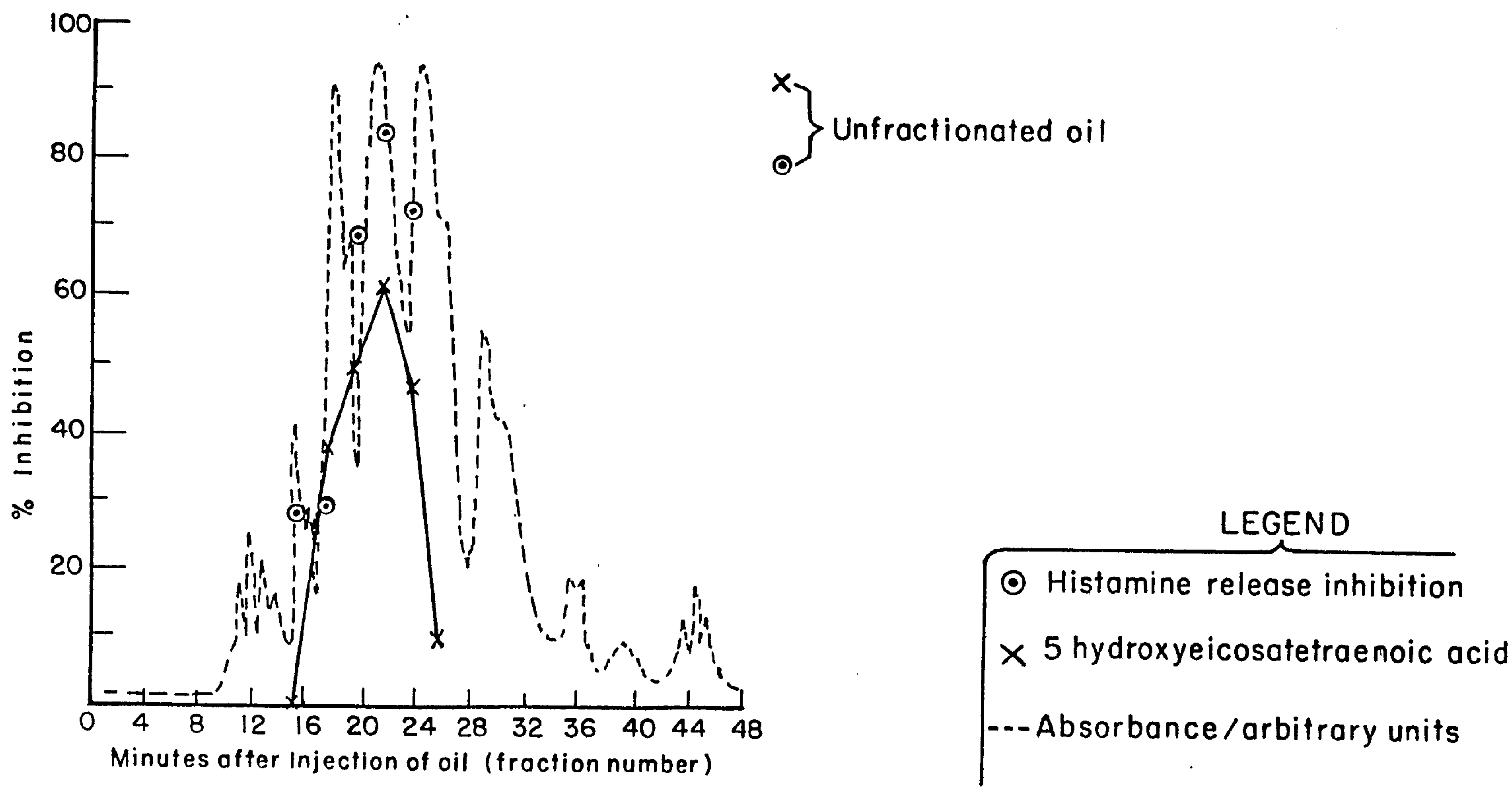
Unfractionated oil
% Inhibition
100
80
60
40
20
0 4 8 12 16 20 24 28 32 36 40 44 48
Minutes after Injection of oil (fraction number)
LEGEND
Histamine release inhibition
5 hydroxyeicosatetraenoic acid
---Absorbance/arbitrary units

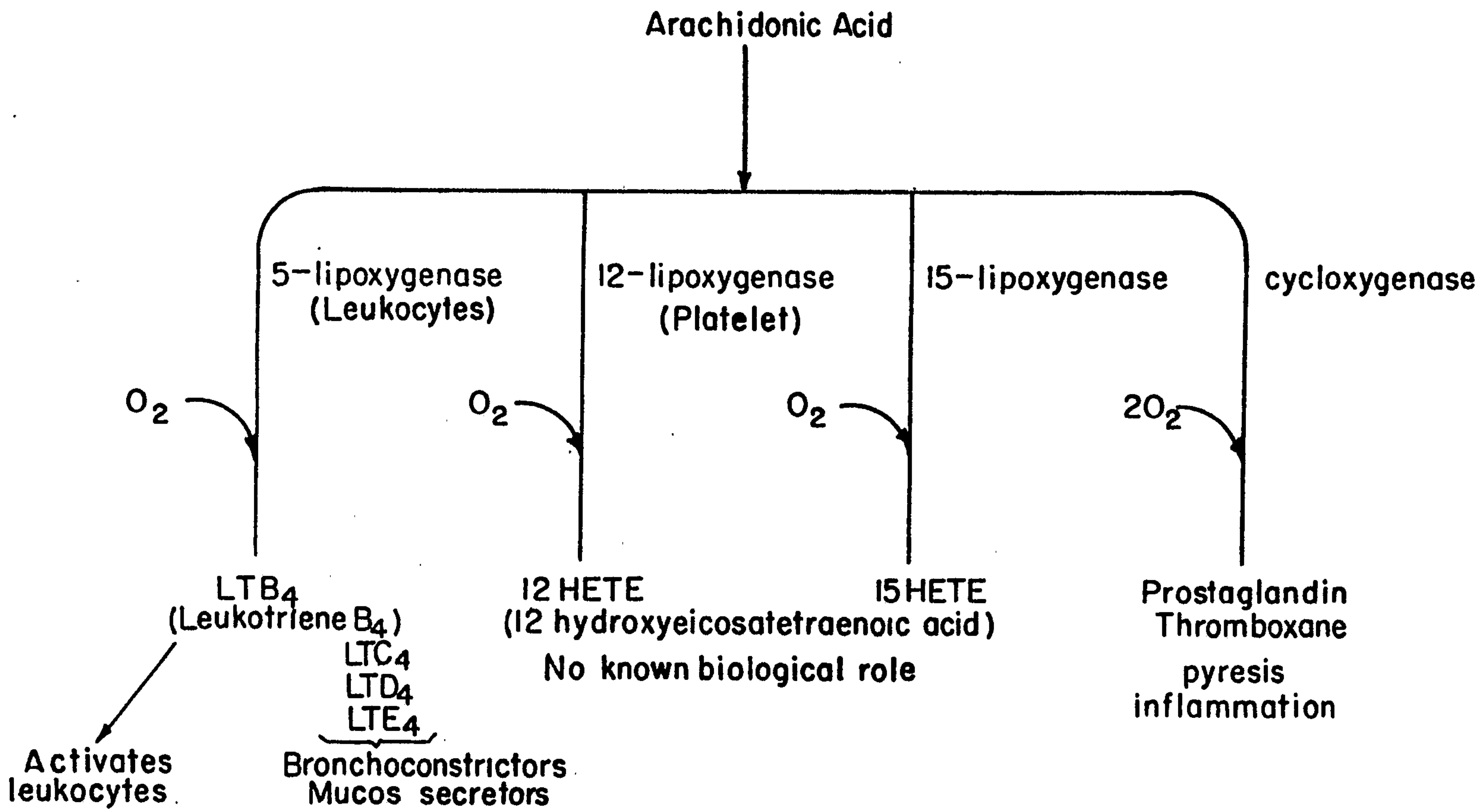
Arachidonic Acid
5-lipoxygenase
(Leukocytes)
12-lipoxygenase
(Platelet)
15-lipoxygenase
cycloxygenase
O2
O2
O2
2O2
LTB4
(Leukotriene B4)
LTC4
LTD4
LTE4
Activates
leukocytes
Bronchoconstrictors
Mucos secretors
12 HETE
15 HETE
(12 hydroxyeicosatetraenoic acid)
No known biological role
Prostaglandin
Thromboxane
pyresis
inflammation

FIG. 2

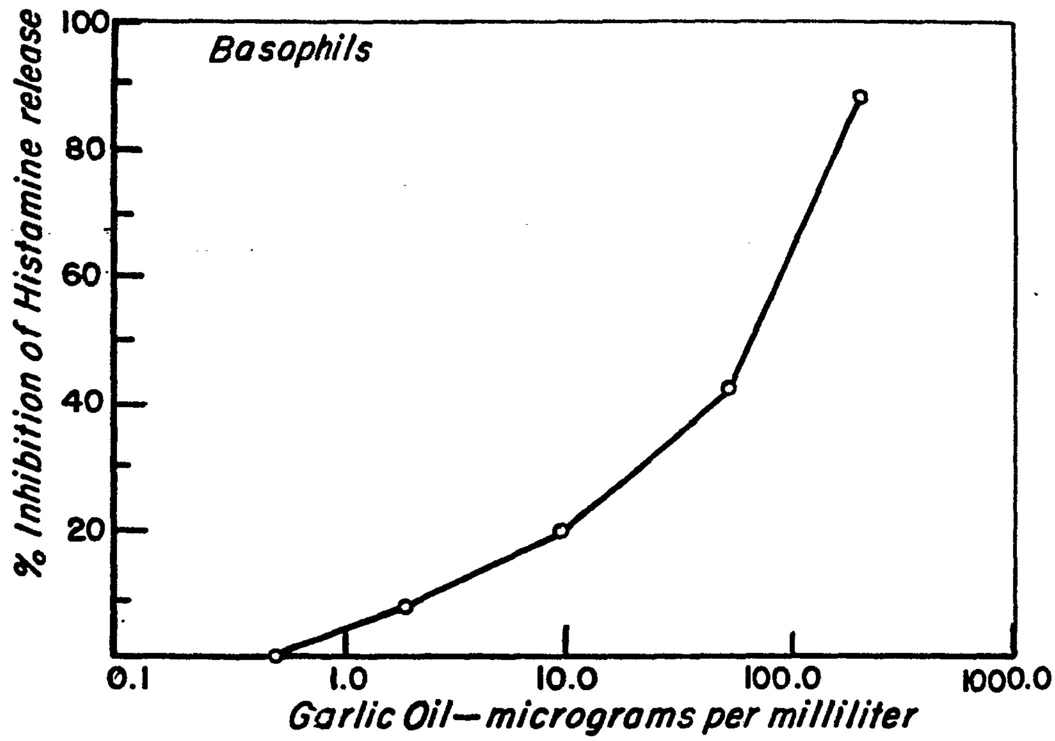
Basophils
% Inhibition of Histamine release
100
80
60
40
20
0.1
1.0
10.0
100.0
1000.0
Garlic Oil—micrograms per milliliter

FIG. 3

FIG. 4

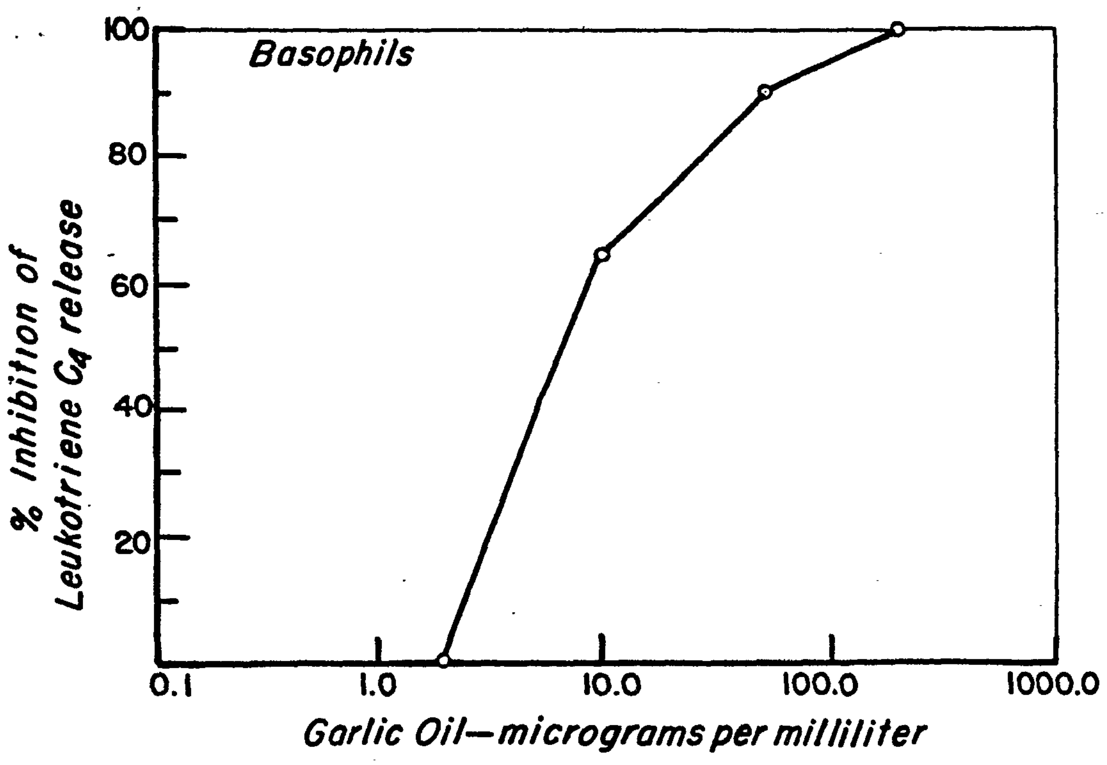
Basophils
% Inhibition of Leukotriene $C_4$ release
100
80
60
40
20
0.1
1.0
10.0
100.0
1000.0
Garlic Oil—micrograms per milliliter

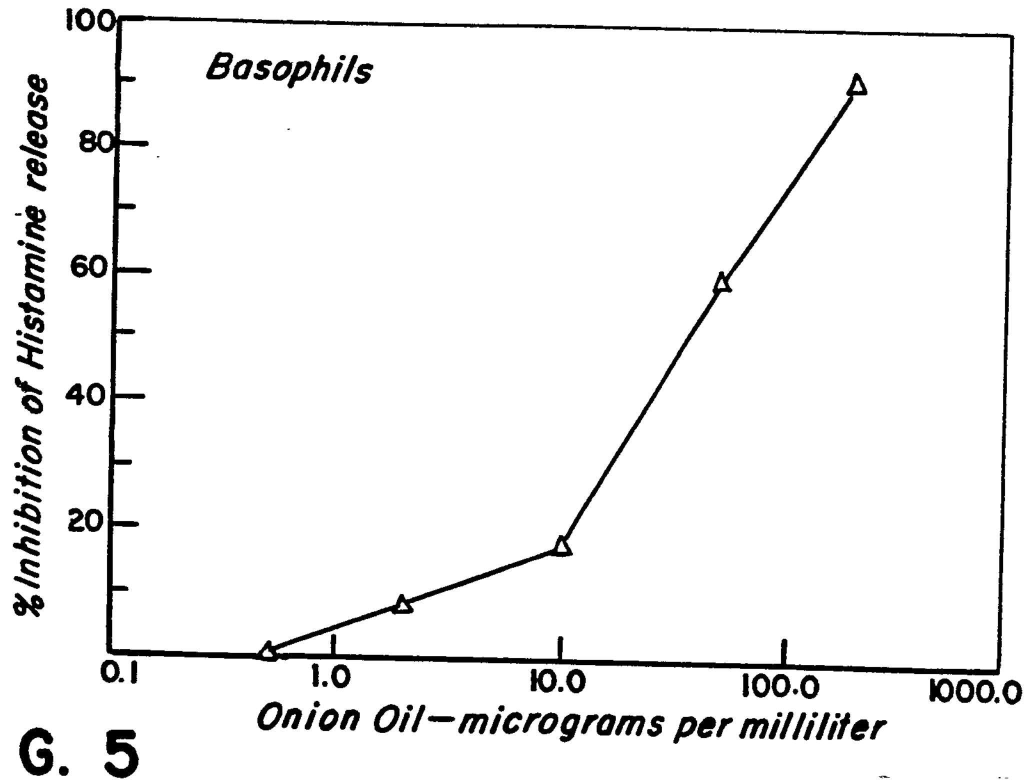
100
80
60
40
20
Basophils
% Inhibition of Histamine release
0.1
1.0
10.0
100.0
1000.0
Onion Oil—micrograms per milliliter

FIG. 5

FIG. 6

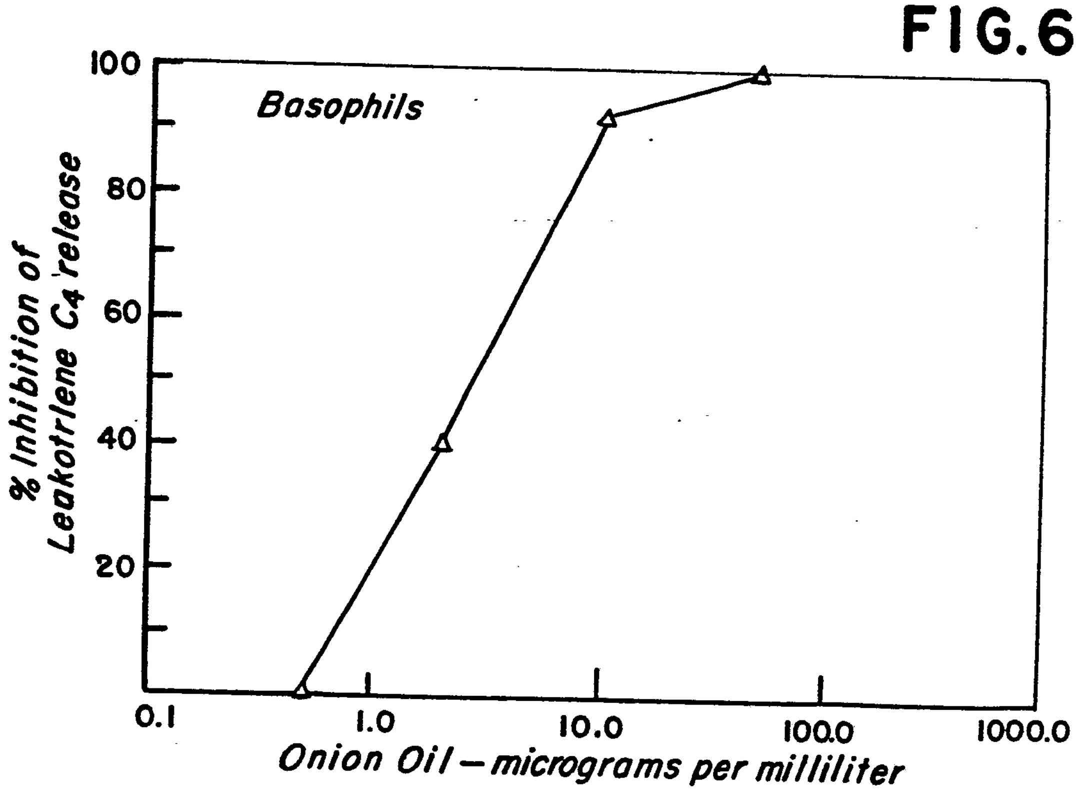
100
80
60
40
20
Basophils
% Inhibition of Leakotriene C4 release
0.1
1.0
10.0
100.0
1000.0
Onion Oil—micrograms per milliliter

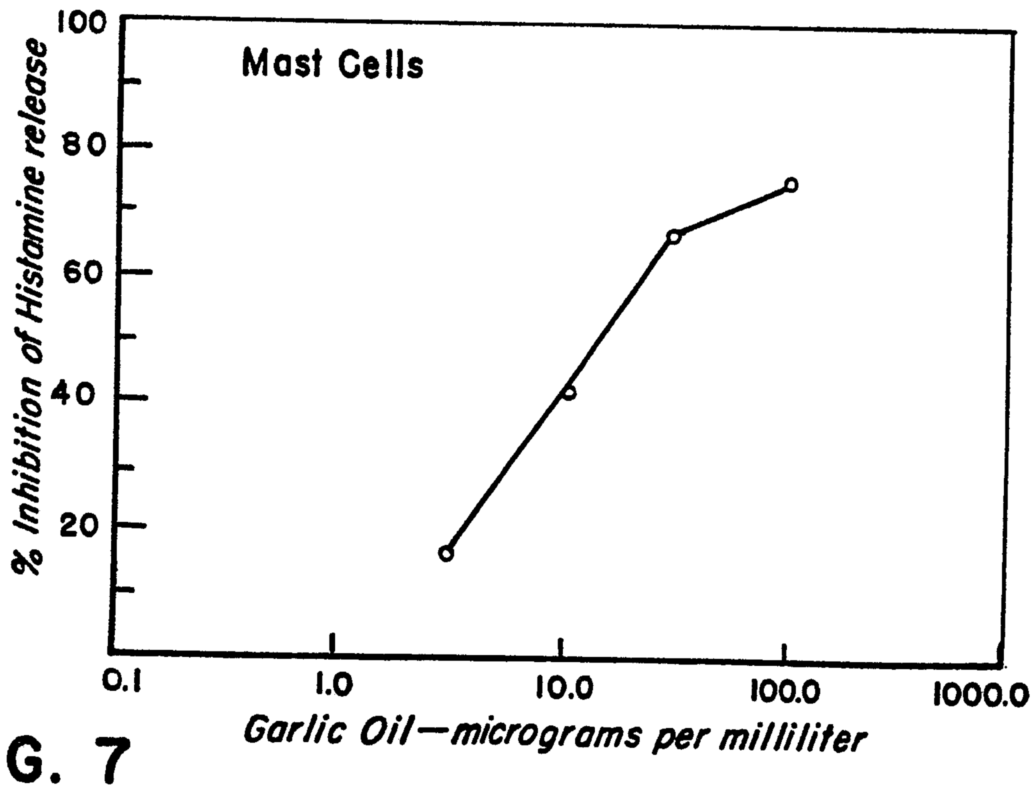
Mast Cells
% Inhibition of Histamine release
100
80
60
40
20
0.1
1.0
10.0
100.0
1000.0
Garlic Oil—micrograms per milliliter

FIG. 7

FIG.8

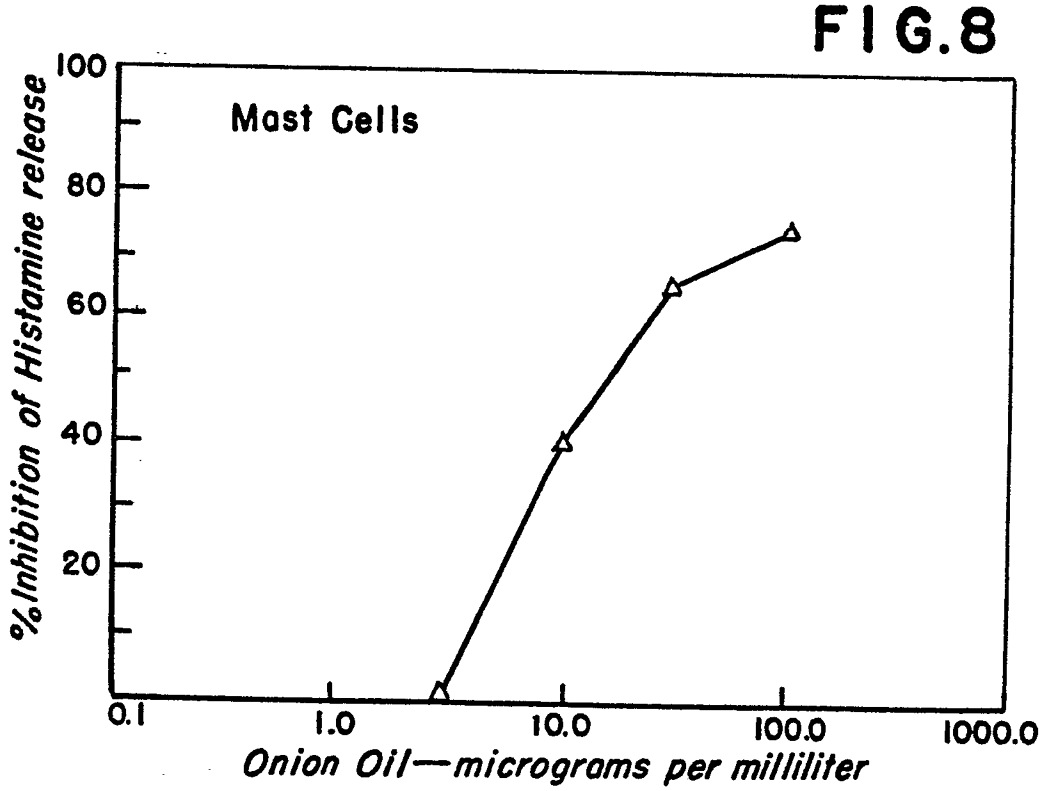
Mast Cells
%Inhibition of Histamine release
100
80
60
40
20
0.1
1.0
10.0
100.0
1000.0
Onion Oil—micrograms per milliliter